(19) Europäisches Patentamt / European Patent Office / Office européen des brevets

(11) **EP 4 218 544 B1**

(12) **EUROPEAN PATENT SPECIFICATION**

(45) Date of publication and mention
of the grant of the patent:
**01.04.2026   Bulletin 2026/14**

(21) Application number: **22205583.2**

(22) Date of filing: **04.11.2022**

(51) International Patent Classification (IPC):
***A61B 3/10*** *(2006.01)*

(52) Cooperative Patent Classification (CPC):
**A61B 3/102**

(54) **APPARATUS AND METHOD FOR IMAGING AN EYE**

VORRICHTUNG UND VERFAHREN ZUR ABBILDUNG EINES AUGES

APPAREIL ET PROCÉDÉ D'IMAGERIE D'UN OEIL

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **12.11.2021   US 202163278609 P
26.10.2022   US 202217973569**

(43) Date of publication of application:
**02.08.2023   Bulletin 2023/31**

(73) Proprietor: **TOPCON CORPORATION
Tokyo 174-8580 (JP)**

(72) Inventors:
• **MINO, Toshihiro
West New York, 07093 (US)**
• **WANG, Yahui
Hoboken, 07030 (US)**
• **WANG, Zhenguo
Ridgewood, 07450 (US)**
• **CHAN, Kinpui
Ridgewood, 07450 (US)**

(74) Representative: **Gagel, Roland
Patentanwalt Dr. Roland Gagel
Landsberger Strasse 480a
81241 München (DE)**

(56) References cited:
**US-A1- 2015 211 838     US-A1- 2016 128 565
US-A1- 2017 105 618     US-A1- 2021 109 340**

## Description

FIELD OF THE INVENTION

The present disclosure relates generally to an apparatus and method for obtaining information concerning a target object, and in particular, an apparatus and method for capturing a data and three-dimensional images of an eye.

BACKGROUND OF INVENTION

The "background" description provided herein is for the purpose of generally presenting the context of the disclosure. Work of the presently named inventors, to the extent it is described in this background section, as well as aspects of the description which may not otherwise qualify as prior art at the time of filing, are neither expressly nor impliedly admitted as prior art against the present invention.

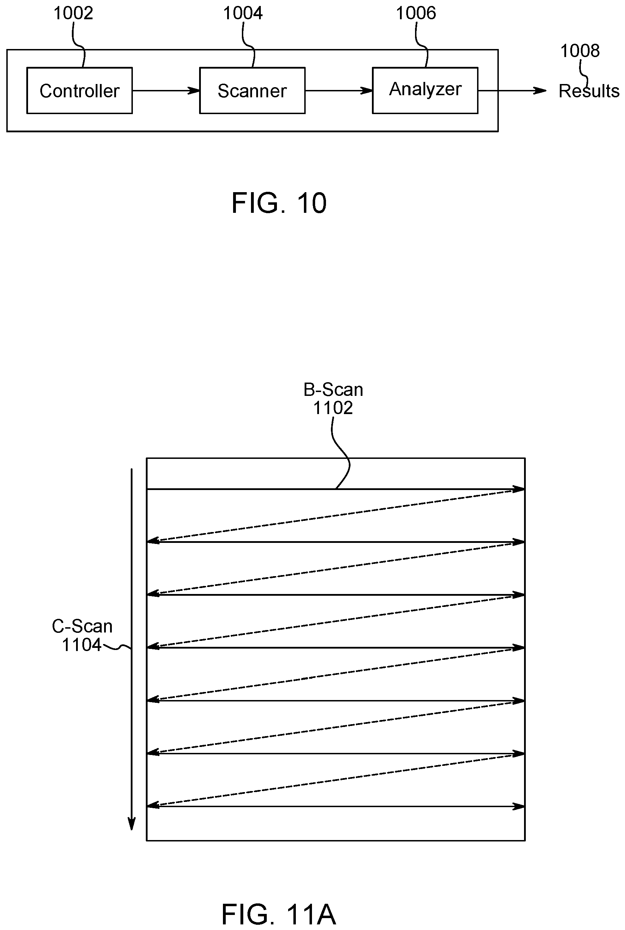

Fig. 10 shows an example of a conventional apparatus for obtaining images of an eye. Such an apparatus includes a scanner 1004 that illuminates the eye and captures raw image data of the eye. The scanner 1004 is controlled by a controller 1002. An analyzer 1006 performs analysis on the raw image data to produce results 1008, such as three-dimensional images.

The scanner 1004 typically moves a scanning spot to a first region to be examined, illuminates the first region with an illumination light, captures data concerning the first region, and then moves the scanning spot to a next region to be examined. The movement path of scanning spot is called a scanning trajectory. The area of the target object imaged in each scanning spot region is relatively small. Typical image spot size is ~20 $\mu$m in ophthalmic imaging. The position of the illuminated spot is scanned by measuring scattered light that covers the imaging target length (area) of at least a few mm. Therefore, the scanning spot is moved from region to region and the data from the regions are combined to thereby obtain data spanning a wider area of the target object.

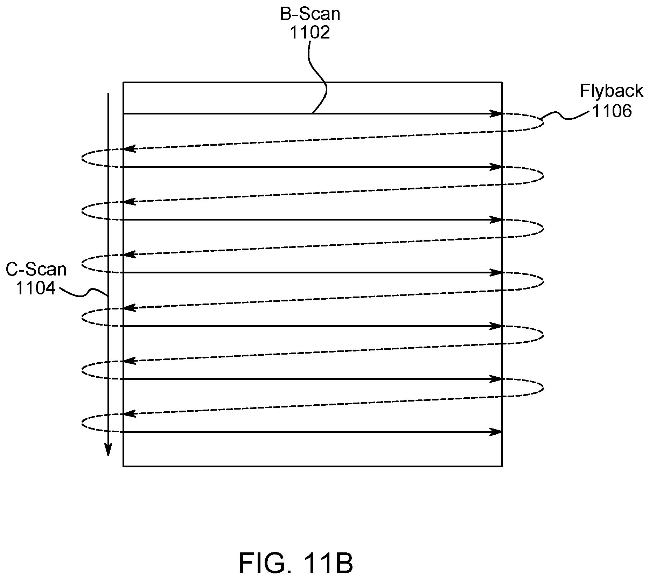

Fig. 11 shows a conventional scanning trajectory used to obtain data regarding a two-dimensional region of a target object. According to this example, a plurality of b-scans 1102 are performed by moving the scanning spot in a left to right direction, and after the scanning spot is moved a full b-scan length to the right-most direction, the scanning spot is moved downward in a c-scan 1104 direction to where a next b-scan 1102 is performed.

However, when the target object is an eye of a living subject, eye movement and fluid movement occur during examination, and therefore, errors are likely to occur when combining the data from different scanning spots.

Document US 2017/105618 A1 relates to optical coherence tomography using spiral scan patterns and square spiral scan patterns.

Document EP 3 021 071 A1 relates to optical coherence tomography using various scan patterns, including, inter alia, hypotrochoidal scan patterns.

Document US 2021/109340 A1 relates to intra-luminal OCT and discloses a cycloid scanning path.

SUMMARY OF INVENTION

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not limited to limitations that solve any or all disadvantages noted in any part of this disclosure.

According to an embodiment of the invention, a scanning apparatus includes the features defined in claim 1.

The * scanning apparatus may be arranged such that each of the plurality of b-scan paths has a same shape and size.

The scanning apparatus is arranged such that each of the plurality of b-scan paths is a closed loop that returns to a starting point.

The scanning apparatus may be arranged such that each of the plurality of b-scan paths is a circular path.

The scanning apparatus is arranged such that the c-scan path is a spiral moving from one of a central region or a peripheral region to the other, or an expanding or contracting rectangular path that moves from one of a central region and a peripheral region to the other.

The scanning apparatus may be arranged such that the area of the target object covered by the movement path is increased in each of two dimensions by increasing a length of the c-scan path.

The scanning apparatus may be arranged such that the controller is further configured to control an inter-scan time by varying a length of each of the b-scan paths and maintaining a sampling step less than a spot size.

The scanning apparatus may be arranged such that the controller is further configured to control the b-scan path to move continuously and with a constant speed in each of x and y directions.

The scanning apparatus may be arranged such that the controller is further configured to control the scanner to perform multiple b-scan paths in a same location before moving the scanning path to a next position along the c-scan path.

The scanning apparatus may be arranged such that the c-scan path is a closed shape.

The scanning apparatus may be arranged such that the b-scan path and the c-scan path are each moved through two or three dimensions.

The scanning apparatus may be arranged such that a start of a previous b-scan is at a first angular position with respect to a center of the previous b-scan; a start of a next b-scan is at a second angular position with respect to a center of the next b-scan; and the first angular position is different than the second angular position.

An embodiment of the invention includes a scanning method as defined in claim 9.

An embodiment of the invention includes a computer readable medium storing computer instructions defined in claim 10.

BRIEF DESCRIPTION OF THE DRAWINGS

The scope of the present disclosure is best understood from the following detailed description of exemplary embodiments when read in conjunction with the accompanying drawings, wherein:

Fig. 1 is a block diagram of an embodiment of the invention;
Fig. 2A is a view of a scanning trajectory according to an embodiment of the invention;
Fig. 2B is another view of a scanning trajectory according to an embodiment of the invention;
Fig. 3A is another view of a scanning trajectory according to an embodiment of the invention;
Fig. 3B is another view of a scanning trajectory according to an embodiment of the invention;
Fig. 4A is an example a scanning trajectory that may produce artifacts according to an embodiment of the invention;
Fig. 4B is an example of a scanning trajectory that may reduce artifacts according to an embodiment of the invention;
Fig. 5 is an example of another scanning trajectory according to an embodiment of the invention; B
Fig. 6A is another view of a scanning trajectory according to an embodiment not forming part of the invention;
Fig. 6B is another view of a scanning trajectory according to an embodiment not forming part of the invention;
Fig. 7 is a block diagram of a method according to an embodiment of the invention;
Fig. 8 is a plot of decorrelation vs. inter-scan time illustrating a method according to an embodiment of the invention;
Fig. 9 is a block diagram of a computer and computer connectivity according to an embodiment of the invention;
Fig. 10 is an example of a conventional apparatus for obtaining images of an eye;
Fig. 11A is an example of a conventional scanning trajectory; and
Fig. 11B is another example of a conventional scanning trajectory.

Further areas of applicability of the present disclosure will become apparent from the detailed description provided hereinafter. It should be understood that the detailed description of exemplary embodiments is intended for illustration purposes only and are, therefore, not intended to necessarily limit the scope of the disclosure.

DETAILED DESCRIPTION

As an area of captured data, or the field of view (FOV), in the scan is increased according to the conventional scanning trajectory in Fig. 11A, the length of each b-scan 1102 increases. Thus, using the conventional scanning trajectory, the length of each b-scan 1102 depends on the desired FOV. Such a conventional scanning trajectory may make multiple passes of each b-scan 1102 to obtain overlapping data, or may move the scanning trajectory in the c-scan 1104 slowly so that scanning spots of a subsequent b-scan 1102 overlaps a previous b-scan 1102. An amount of time that elapses between successive scans of an overlapping area on the target object is referred to as inter-scan time.

As discussed further below, it may be useful to control the inter-scan time. However, according to the conventional scanning trajectory in Fig. 11, the inter-scan time also increases with FOV.

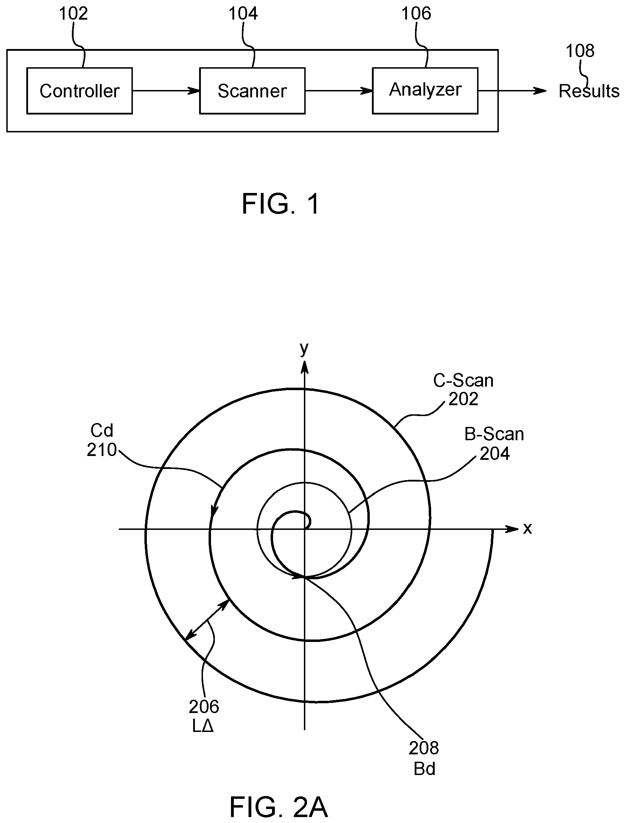

Fig. 1 shows an embodiment of an apparatus according to the invention. The apparatus includes a controller 102, a scanner 104, and an analyzer 106 to produce results 108. The controller 102 includes one or more computers or programmed processors and/or hardware circuits configured to control the scanner 104. The scanner 104 includes light illumination and sensing devices that control movement of a scanning spot along a scanning trajectory to thereby capture data concerning a region of a target object. The scanner may operate to collect two-dimensional or three-dimensional data regarding the target object. The scanner 104 may include an Optical Coherence Tomography (OCT) scanner. Two-axis galvanometer scanners are typically used in OCT system. The analyzer 106 may include one or more computers or programmed processors and/or hardware circuits configured to receive the results of scanning (e.g., OCT scan data) and produce results 108 based on the scanning results. The results 108 may include two-dimensional or three-dimensional graphical visualizations of the target object, and/or quantitative information concerning the target object (e.g., blood flow speed).

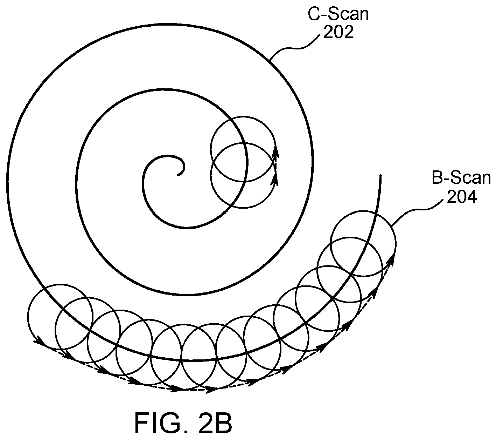

Fig. 2A shows a scanning trajectory according to an embodiment of the invention and including one or more circular path b-scan 204 and a spiral path c-scan 202. In this example, a first circular b-scan 204 is performed in the Bd 208 direction (i.e., counterclockwise) with a center of the b-scan 204 circle near a center of a two-dimensional (x, y) target area to be observed. After the first circular b-scan 204 is completed, a second circular b-scan 204 may be performed centered on a second position arranged outwardly (i.e., along the Cd 210 direction) along the spiral c-scan 202 path. The distance moved between successive b-scan circle centers are described by $L_c/N_c$, where $L_c$ is c-scan length and $N_c$ is sampling points of c-scans. A distance between adjacent arms of the spiral is $L_\Delta$, which is related redundancy. Alternatively, two or more b-scans may be performed in a same or similar location before moving to a next location. Fig. 2B shows another view of the scanning trajectory to illustrate additional b-scans 204.

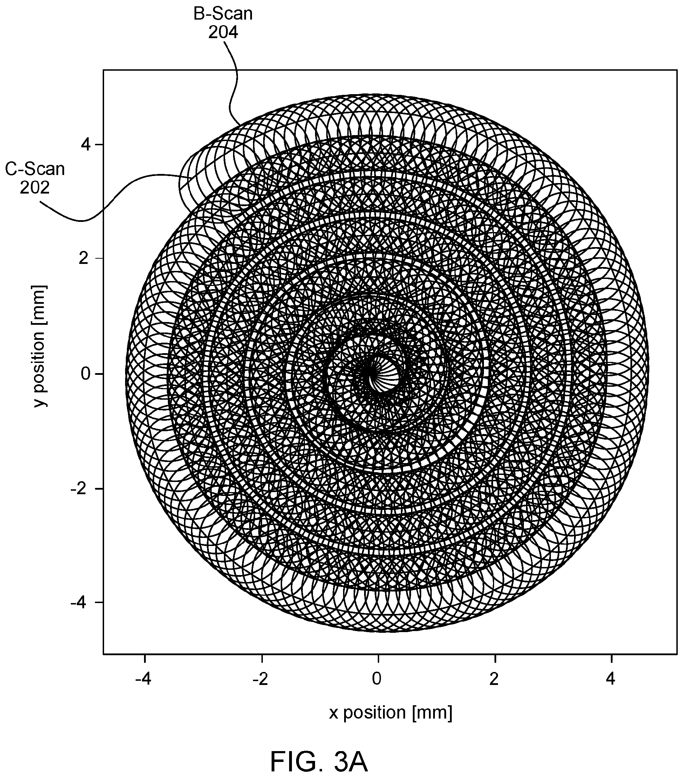

Fig. 3A is another view of the scanning trajectory in Figs. 2A and 2B, showing plural b-scans 204 along a spiral path c-scan 202, and indicating typical x and y axis dimensions. The scanning pattern trajectory in this example is described by the following equations:

$$\mathrm{x}(i_b, i_c) = -\frac{L_b}{2\pi}\cos\left(\frac{2\pi}{N_b}i_b\right) + \sqrt{\frac{L_c L_\Delta}{\pi N_c}i_c}\cos\left(\sqrt{\frac{4\pi L_c}{L_\Delta N_c}i_c}\right)$$

$$\mathrm{y}(i_b, i_c) = -\frac{L_b}{2\pi}\sin\left(\frac{2\pi}{N_b}i_b\right) + \sqrt{\frac{L_c L_\Delta}{\pi N_c}i_c}\sin\left(\sqrt{\frac{4\pi L_c}{L_\Delta N_c}i_c}\right)$$

$L_b$ and $L_c$ represent lengths of the b-scan 204 and c-scan 202, $N_b$ and $N_c$ are sampling points of b-scans 204 and c-scans 202. $i_b$ and $i_c$ represents $i^{th}$ b-scan 204 and c-scan 202. $L_\Delta$ indicates gap between adjacent spiral trajectory and is related to redundancy. Fig. 3A illustrates an exemplary scanning trajectory in the case of $L_b$ = 4.5 mm, $L_c$ = 72 mm, $L_\Delta$ = 0.4 mm, $N_b$ = 51, $N_c$ = 410. For this illustration, $N_b$ and $N_c$ are set to extremely small number (~1/10) compared to actual cases for better visualization of scanning trajectory. The invention also includes other possible values of the variables, depending upon data capture requirements. Since it may be preferred to keep sampling density along b-scan and c-scan similar, the ratio between $L_b/N_b$ (b-scan sampling density) and $L_c/N_c$ (c-scan sampling density) is typically less than 3. As shown by this example, redundancy may be less at an outermost area and at a center of the spiral trajectory (i.e., beginning of scan).

The inventive scan trajectory may provide several advantages over a conventional scan trajectory.

First, a conventional scan trajectory may include a raster-like motion of the scanning position. Fig. 11A shows an idealized view of a conventional scan trajectory. Fig. 11B shows a more realistic view illustrating one problem with the conventional scan trajectory. According to the example in Figs. 11A and 11B, after each completed b-scan 204 in a left-to-right direction, the scanning position must be moved in an almost opposite right-to-left direction. Scanner 1004, for example, using galvo-scanner technology, cannot perform such a change in direction immediately, and therefore, there is some time delay for the change in direction that results in reduced duty ratio of the scan. As shown in Fig. 11B, the conventional raster scan has flyback 1106 during which the scanner drives the scanning position back to the beginning of a next b-scan 1102. Duty ratio is defined by time corresponding to the dotted line divided by total scan time.

Second, in a conventional scan trajectory, the b-scan 204 proceeds in only a single, left-to-right direction (i.e., x direction). However, according to the inventive scan trajectory, during each circular b-scan 204 the scanning position moves continuously in both x and y directions. Thus, the scanner may be driven by a simple sine function and the component elements of the scanner (i.e., an x-direction scanner and y-direction scanner) may advantageously be driven simultaneously at a constant speed. Constant speed motion allows the inventive scan trajectory to have an easily controlled sampling density. By reducing computational cost, constant speed motion also simplifies application of computational adaptive optics (CAO), which is otherwise computationally expensive. This is an advantage compared to other scanning trajectories, for example, a Lissajous shaped scan path. Scan speed of effective area of b-scan 1102 is usually constant in a conventional raster scan trajectory.

Third, since each b-scan 204 according to the inventive scan trajectory completes close to the starting point of the next b-scan, there is less time spent repositioning to the start of the next b-scan 204. Thus, an embodiment of the invention may also advantageously keep duty ratio high.

Fourth, according to the inventive scan trajectory, inter-scan time is primarily a function of b-scan length $L_b$ and b-scan speed. Although inter-scan time is also a function of b-scan length in the conventional raster scan trajectory, the inventive scan trajectory can achieve a shorter inter-scan time due to the high duty ratio (close to 100%). Also, the inventive scan trajectory allows for control of the inter-scan time without limiting FOV (as discussed further below). Thus, inter-scan time may be adjusted without changing the coverage area. As discussed further below, Variable Inter Scan Time Analysis (VISTA) may be performed, according to another embodiment of the invention, by varying the inter-scan time, may be made more convenient and repeatable by the inventive scan trajectory. Also, according to the inventive scan trajectory it is possible to make inter-scan time longer and thereby improve signal contrast in Optical Coherence Tomography Angiography (OCTA).

Another way to control inter-scan time using a conventional scan trajectory is by changing sampling density (namely scan speed). However, in OCTA where inter-scan time is an important parameter, high sampling density (i.e., sampling step is about half of spot size) is required to visualize fine structure of a vessel, and as a result, there is not much available room for adjustment of sampling density in practice using the conventional scan trajectory. So, the inventive scan trajectory may be able to achieve better results by controlling inter-scan time by varying a length of each b-scan while keeping FOV and sampling step less than spot size (i.e., high sampling density), which is not possible with the conventional scan trajectory. Spot size is defined by size of illumination light for OCT. Sampling density means how many data points are measured per unit length. Higher sampling density means that more data points are measured per unit length. Sampling step is how much length is moved from a current measurement position to perform a measurement at the next b-scan point. Sampling density is therefore inverse of sampling step.

Fifth, according to the inventive scan trajectory, the FOV is independent of b-scan length $L_b$. Thus, the FOV may be increased by merely extending the c-scan length $L_c$. In other words, by merely extending the length (duration) of the spiral c-scan, the field of view may be increased. Such an increase also advantageously does not result in a change in the inter-scan time.

Sixth, scan overlaps (including b-scan to adjacent b-scan overlap, as well as overlap of a b-scan on one spiral arm to b-scan on a successive or previous spiral arms) can be used for motion estimation and correction. For example, when calculating correlation among OCT data by using the overlapped area, an embodiment of the invention can obtain the best transformation (shift and rotation etc.) which minimizes the difference or error due to eye motion.

Although the inventive example above is illustrated with a circular b-scan 204, the invention encompasses any closed b-scan shape (i.e., where each b-scan path returns to a same or approximately same starting point), including but not limited to oval, elliptical, and lemniscate paths.

Further, although the inventive example above is illustrated with each b-scan 204 including only a single pass over the covered region, the invention also includes repeating each b-scan 204 in the same location, or in a slightly adjusted location, for more than one pass.

In addition, although the inventive example above is illustrated with the movements in each of the b-scan 204 and c-scan 202 being in a same x,y plane, the invention also includes moving the b-scan 204 and c-scan 202 through different planes. Further, the invention includes each of the b-scan 204 and c-scan 202 moving independently in three dimensions.

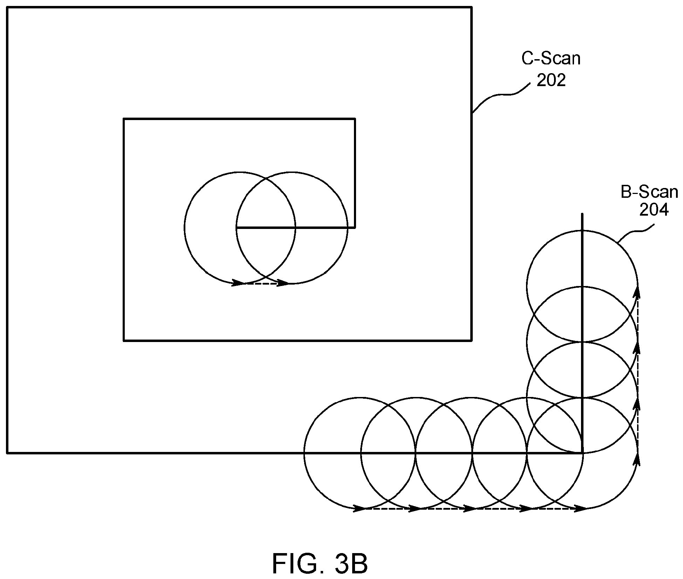

Further, although the inventive embodiment is shown having a c-scan path that is a spiral moving from a central region to a peripheral region, the invention also includes a spiral c-scan path that moves from a peripheral region to a central region. Also, the invention includes other c-scan paths that move from one of a central region or a peripheral region to the other of the central region or a peripheral region (e.g., an expanding or contracting rectangular path), for example as shown in Fig. 3B. The inventive c-scan path may also be any continuous path within the field of view.

As an alternative embodiment, the invention includes a continuous shape b-scan rather than discrete b-scans. That is, the plural b-scans 204 shown in Fig. 3A are alternatively constructed as an uninterrupted path that continuously moves along the c-scan direction.

The inventive scan pattern may be applied to various scanning technologies including OCT scanning, OCTA, and VISTA. When performing OCTA, differences between OCT scans performed at different times are used to detect fluid flows in a living body. VISTA is a method to obtain additional information about the fluid flows, for example, as discussed further below.

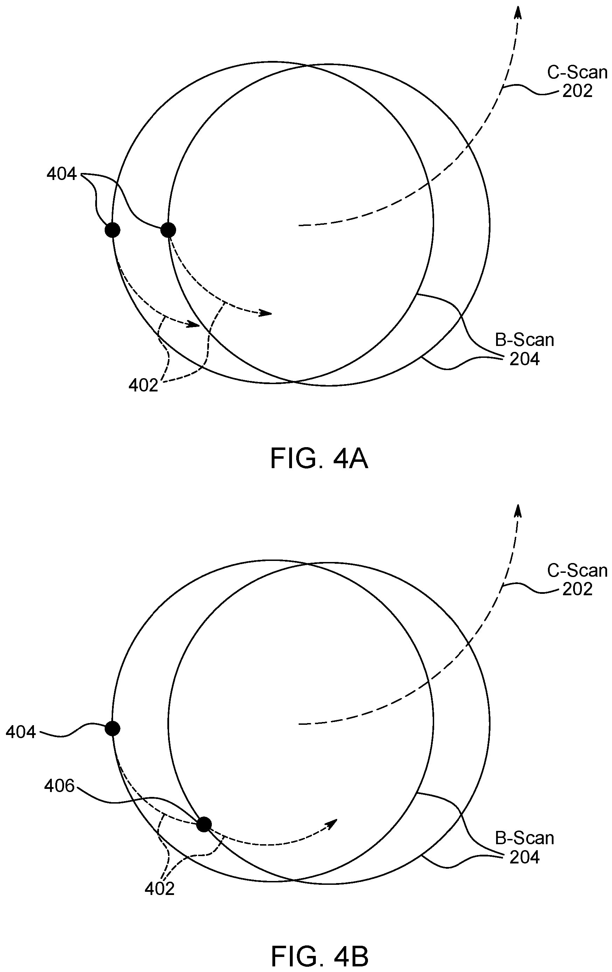

When the inventive scanning trajectory noted above is applied to repeated scans used for OCTA, artifacts may appear at the beginning of each b-scan. For example, as illustrated in Fig. 4A, with an idealized scanner mechanism, each b-scan 204 along the c-scan path 202 would start at a same angular location 404 with respect to a center of the b-scan path 202, and with no transition time. However, in a practical scanner, the movement of the scanning position from one b-scan path 204 to a next b-scan path requires a transition path 402. If data is continuously collected during the transition 402, there is a greater chance of OCTA artifacts, since the transition path 402 does not match the b-scan 204 path.

To reduce the capture of artifacts, an alternative inventive embodiment shown in Fig. 4B which disables data collection during the transition 402 from one b-scan 204 to the next b-scan 204. Thus, the start of data collection in a subsequent b-scan path 204 is shifted to position 406, and the start of data collection of the subsequent b-scan path is at a different angular location 406 with respect to a center of the subsequent b-scan path than an angular location 404 of the start of data collection of the previous b-scan path 204. This alternative embodiment has a side effect of slightly reducing duty ratio, while advantageously eliminating the artifact.

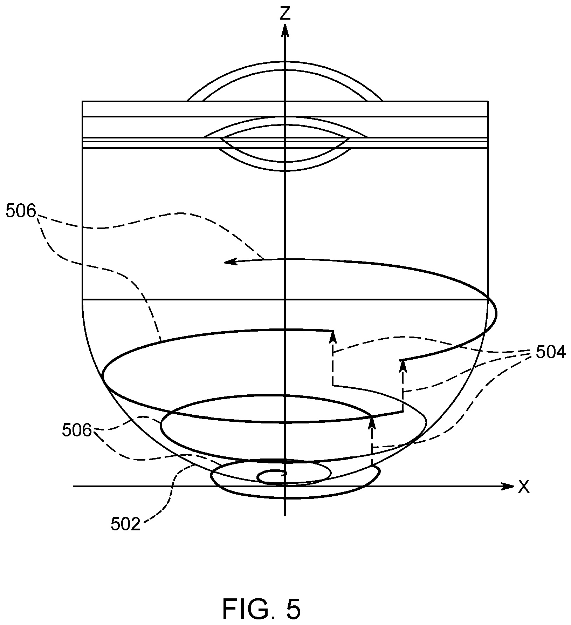

Fig. 5 is an example of an alternative embodiment of the scan path in which the z-axis direction (along a direction from the scanner to the target object, e.g., in and out of an eye) of the spiral c-scan path 506 may be varied. Particularly when the target object is a curved retinal surface 502 of an eye, the c-scan location may be changed in the z-direction at discrete instances 504 at locations along the scan path 506 to thereby allow the scan pattern to follow the contour of the target object. Thus, the inventive alternative embodiment may help to achieve wide-field OCT by avoiding defocus and image flipping due to limited imaging depth range of OCT.

The inventive scan trajectory can realize wide FOV while achieving relatively shot inter-scan time, which is advantageous for application to VISTA. For example, VISTA may be performed using a b-scan length of 4.5 mm (512 pixels), corresponding to approximately 1.4 mm diameter, and a c-scan length of 25.3 mm (1920 pixels), and $L_\Delta = 0.88$ mm, with 4 repetitions.

OCTA scanning may require high quality data collection in a limited FOV. To achieve that, another embodiment of the invention may use an ellipsoidal c-scan path instead of the spiral scan path discussed above.

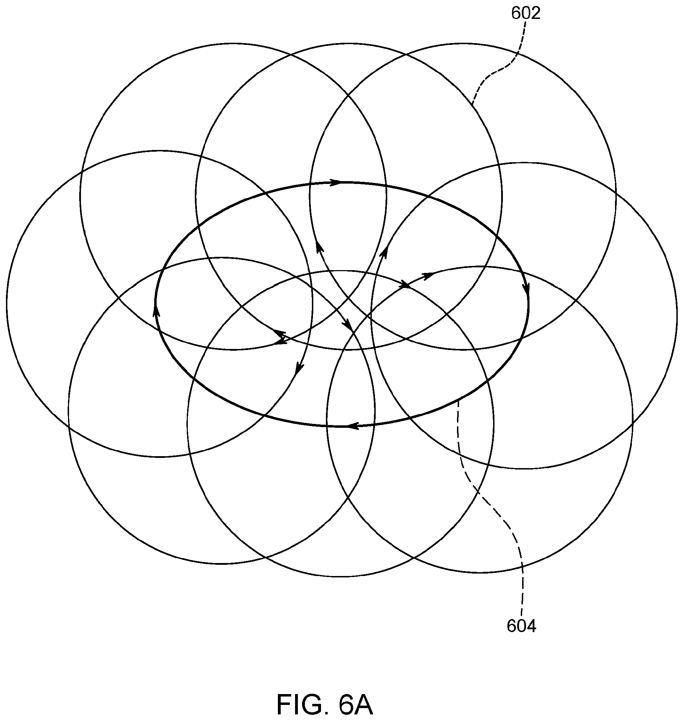

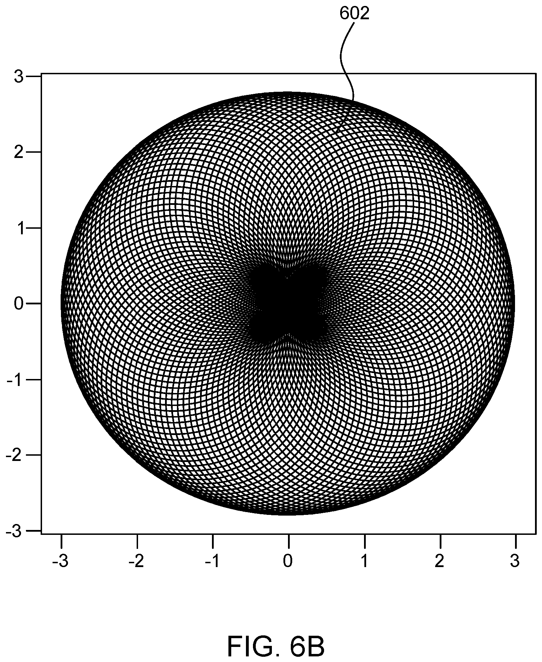

Figs. 6A and 6B show an example of another "cycle-scan" trajectory embodiment not forming part of the invention in which the b-scan 602 is circular and the c-scan 604 is a closed ellipsoidal path.

Trajectory of the cycle scan trajectory according to this embodiment is given by the following equations:

$$x(i_b, i_c) = \frac{L_b}{2\pi} \cos\left(\frac{2\pi}{N_b} i_b\right) + \cos\left(\frac{2\pi}{n_c} i_c\right) \left\{ \left(\frac{\sin\left(\frac{2\pi}{n_c} i_c\right)}{\frac{L_b}{2\pi} + \Delta}\right)^2 + \left(\frac{\cos\left(\frac{2\pi}{n_c} i_c\right)}{\frac{L_b}{2\pi} - \Delta}\right)^2 \right\}^{-0.5}$$

$$y(i_b, i_c) = \frac{L_b}{2\pi} \sin\left(\frac{2\pi}{N_b} i_b\right) + \sin\left(\frac{2\pi}{n_c} i_c\right) \left\{ \left(\frac{\sin\left(\frac{2\pi}{n_c} i_c\right)}{\frac{L_b}{2\pi} + \Delta}\right)^2 + \left(\frac{\cos\left(\frac{2\pi}{n_c} i_c\right)}{\frac{L_b}{2\pi} - \Delta}\right)^2 \right\}^{-0.5}$$

$L_b$ and $N_b$ represent length and sampling points of the b-scan 602. $i_b$ and $i_c$ indicate $i^{th}$ b-scan 602 and c-scan 604. $n_c$ is number of sampling points for 1 cycle of c-scan 604 and $\Delta$ is difference between longer and shorter axis length of ellipse.

The c-scan 604 may be iterated for more than one cycle to guarantee acquiring effective data at every position.

A typical OCTA scan according to the cycle-scan trajectory embodiment may include a b-scan length of 9 mm (1024 pixels), corresponding to 2.8 mm diameter, $\Delta = 0.1$ mm, repetitions = 2, and c-scan length of 1888 pixels, with a cycle size of 384 pixels (iteration 4.9 times). "Repetition" refers to how many times a b-scan is repeated to get an OCTA signal as discussed above. "Iteration" is how many times a circle c-scan is repeated. "Cycle size" is how many c-scan points are measured in 1 iteration (i.e., in one circle c-scan).

Voluntary eye movement and blinking may occur during examination. Since each location is measured at well dispersed timing, a reconstruction algorithm can successfully generate completed data by using only effective data, and there is no missing coverage areas according to this embodiment.

By reducing cycle size and scan area in cycle trajectory scan, OCT/OCT volume data can be acquired in a short time (i.e., even less than 0.1 msec) with correcting voluntary eye movement. Cycle trajectory scan according to the embodiment may also realize temporal analysis of OCT/OCTA including dynamic contrast technique. Dynamic contrast is a technique to determine contrast based on temporal change and to provide images where contrast reflects temporal change. Temporal analysis is a technique for analyzing change in data along the time axis.

Although the example of Figs. 6A and 6B are shown with an elliptical c-scan path, the invention does not include using any closed path for the c-scan, such as, but not limited to circular, oval, lemniscate, square, closed polygon paths, or any hybrid mixture thereof.

Sampling density can impact OCTA. For example, even when varying sampling density along c-scan between 13.2 μm, 19.6 μm, and 26.4 μm, with sampling density along b-scan of 8.8 μm it is found that b-scan sampling density is more important factor than c-scan sampling density in determining OCTA image quality. Different from a conventional raster scan trajectory, reducing c-scan sampling density using the inventive trajectory does not impact to signal of high spatial frequency in a certain direction, and scanning can be performed using dense b-scan sampling and sparse c-scan sampling without obvious impact on image quality.

Further, according to the embodiments, redundancy (overlapping) is prioritized rather than c-scan sampling density in the scan design, which is effective to stabilize motion correction and reduce importance of each a-scan data. That is, information provided by one a-scan data can be covered by another a-scan data. A-scan data is data from measuring an axial profile (i.e., z-axis) of a target object (eye). Typical scan values may include the following:

$$L_b = \frac{8.8 N_b}{1000} mm, \quad L_c = \frac{26.4 N_c}{1000} mm, \quad L_\Delta = \frac{5 L_b}{9\pi}$$

Conventionally, VISTA is a visualization technology used to graphically illustrate fluid displacement. VISTA utilizes properties of OCTA signal changes over time depending on flow speed and requires multiple repeats scans. OCTA inter-scan time is must faster than used in conventional OCT systems. While OCTA may merely show the difference between continuously acquired OCT images, e.g., for purposes of contrast enhancement, VISTA attempts to graphically represent the relative flow speeds of fluid.

According to a conventional VISTA visualization approach, a composite image of vascular eye structure may be presented with hue or color representing a ratio of OCTA signal collected with shorter inter-scan times vs. longer inter-scan times. The ratio corresponds to an inverse of the saturation time under an assumption that OCTA signals are saturated in longer inter-scan times and linearly increases with displacement between OCT frames. Thus, if we assume that OCTA signal is not saturated yet at shorter inter-scan time but saturated at the longer inter-scan time (under linear model), ratio and inverse of the saturation time should be same thing. For example, a conventional VISTA approach may gradually increase an inter-scan duration in successive scans, and then find a ratio between captured fluid positions of different images to graphically characterize the relative flow speeds. Although the conventional VISTA visualization provides a qualitative view of fluid flow, it does not provide any quantitative assessment of flow rates.

A basic concept of an improved quantitative VISTA according to another embodiment of the invention, is that OCTA signal increases with displacement between OCT frames and saturates at certain amounts of displacement. By finding a time when the OCTA signal becomes saturated (i.e., Tsaturation), an improved quantitative VISTA embodiment of the invention can quantitatively estimate flow speed, rather than merely providing a qualitative visualization. Such a quantitative estimate may be useful for clinical purposes.

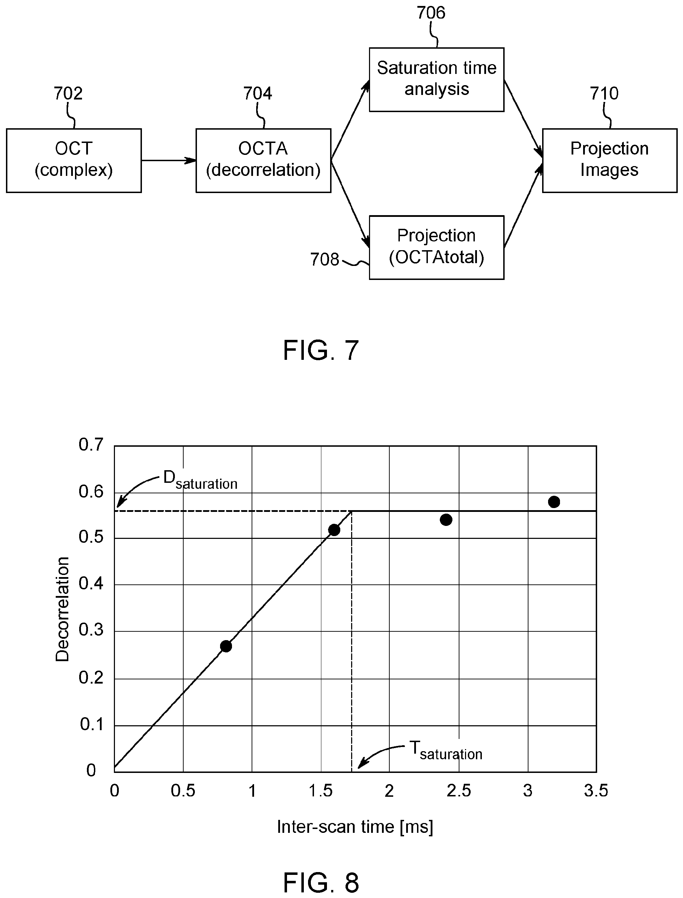

Fig. 7 shows a flow diagram of processes used by an embodiment of the invention to perform quantitative VISTA. First, OCT (complex) is performed in step 702, which includes generating OCT data (spatial domain) from interference data (spectral domain). Next, in step 704, the OCTA data is generated by calculating decorrelation among OCT data acquired by repeated b-scans.

Step 706 includes a saturation time analysis performed for each OCT capture having a different inter-scan time. First, this step assumes that decorrelation increases linearly until decorrelation saturates, as shown by the following equation:

$$\text{decorrelation}_{as}(\tau; x, z)$$

$$= \begin{cases} D_{saturation}/T_{saturation} & \tau = a\tau \ (until \ decorrelation \ is \ sturated) \\ D_{saturation} & (after \ decorrelation \ is \ saturate) \end{cases}$$

Saturation time and saturation decorrelation are determined so that least square error $\delta$ is minimized. Least square error is calculated by the following equation:

$$\delta(x, z) = \sum_{\tau} \left\{ \text{decorrelation}_f(\tau; x, z) - \text{decorrelation}_{as}(\tau; x, z) \right\}^2$$

Then in the case where $\delta(x, z)$ is minimized, differential of the equation is

$$\frac{\partial \delta(x, z)}{\partial a} = 2 \sum_{\tau}^{not \ saturated} \left\{ \text{decorrelation}_f(\tau; x, z) - \text{decorrelation}_{as}(\tau; x, z) \right\} \tau = 0$$

$$\frac{\partial \delta(x, z)}{\partial D_{saturation}} = 2 \sum_{\tau}^{saturated} \left\{ \text{decorrelation}_f(\tau; x, z) - \text{decorrelation}_{as}(\tau; x, z) \right\} = 0$$

Therefore, $D_{saturation}$ and $T_{saturation}$ are calculated according to the embodiment by the following equations.

$$D_{saturation} = \frac{\sum_{\tau}^{saturated} \text{decorrelation}_f(\tau; x, z)}{\sum_{\tau}^{saturated} 1}$$

$$T_{saturation} = D_{saturation} \frac{\sum_\tau^{not\ saturated} \tau^2}{\sum_\tau^{not\ saturated} \text{decorrelation}_f(\tau; x, z)\tau}$$

As shown in Fig. 8, Dsaturation corresponds to the decorrelation value at which the data becomes saturated, and Tsaturation corresponds to an inter-scan time at which the data becomes saturated. As further illustrated by the example of Fig. 8, Tsaturation corresponds to a bend in a curve fitted to plotted points of decorrelation vs. inter-scan time. The inter-scan time at which the curve bends corresponds to the time after which the decorrelation values no longer increase (or increases at a lower rate), with increasing inter-scan time.

Instead of decorrelation, the Tsaturation calculations above may be equivalently performed to find Tsaturation and Dsaturation using any OCTA signal that represents a difference between successive OCT scans (e.g., optical microangiography (OMAG), speckle variance, phase variance, and/or split-spectrum amplitude-decorrelation angiography (SSADA)).

A least square error analysis performed as above for every case where decorrelation saturates between each measurement points yields the condition minimizing least square error (i.e., the condition when the differential of the equation below becomes 0. $\frac{1}{T_{saturation}}$ is then calculated as a parameter quantitively indicating flow speed. Since analysis may not be properly done when decorrelation is too small, the following constraint is also applied:

$$\frac{1}{T_{saturation}} = 0 \ (\text{if } D_{saturation} < 0.1)$$

Thus, saturation time Tsaturation can be determined at each point throughout the three-dimensional data set acquired by OCT scanning, and volume VISTA data is constructed from VISTA b-frame images. VISTA b-frame images are generated by protocol explained below using saturation time images and OCTA images.

Fig. 8 shows an example of using the above calculations and linear model to obtain quantitative values of Tsaturation and Dsaturation, while excluding data points where Dsaturation < 0.1 (i.e., assumed not to have flow).

Although all data points are used in the analysis above for saturation time analysis, an alternative embodiment of the inventive method avoids using decorrelation signal generated by a one pair of OCT data because of the low SNR of decorrelation. For example, according to an embodiment of the invention, it is not necessary to obtain four data points, as in the example of Fig. 8, to perform curve fitting and find saturation time. Since there are two unknown parameters (i.e., Tsaturation and Dsaturation), a minimum required number of data points is only two points. However, if all the data points are equally reliable, using all the data points should provide better results. If reliability of data is different among the data points, fitting may be performed by using a weight reflecting the relative reliability of each data point. In an extreme case where the last data point is not reliable, an alternative embodiment my discard the less reliable last data point and perform fitting without it, which can provide more reliable analysis results of saturation time.

Step 708 preforms projection of OCTA total. For example, the projection image may be an image in the xy-plane (e.g., from a top view) and calculated by compressing data (e.g., averaging or taking maximum etc.) along the z-axis. For example, in order to extract information only from vessel areas, projected image $P(x)$ of data $V(x, z)$ by weighting total OCTA value $OCTA(x, z)$ is calculated as

$$P(x) = \frac{\sum_z V(x, z) \times OCTA(x, z)}{\sum_z OCTA(x, z)}$$

$OCTA(x, z)$ represents ratio of flowing particles under the assumption that OCTA signal is saturated, and it can be considered as probability of vessel.

Step 710 provides composite VISTA projection images based on the OCTA total projection of step 708 and the saturation time analysis of step 706. A projection image of OCTA is used to determine brightness in VISTA composite image and is constructed by taking log to enhance visibility of capillaries. OCTA is generated by calculating decorrelation.

In particular, a VISTA composite image is generated from an OCTA image and an inverse of saturation time image for better interpretation of VISTA result.

Brightness of a VISTA composite image is determined based on OCTA intensity. Black (0) and white (255) may be defined as pixel intensity of OCTA of 5% and 95% quantiles. Brightness of VISTA composite image may be linearly mapped between the black and white level. Namely

$$\text{brighness} = \begin{cases} 0 \ (OCTA < OCTA_{5\%}) \\ 255 \ (OCTA > OCTA_{95\%}) \\ \dfrac{OCTA - OCTA_{5\%}}{OCTA_{95\%} - OCTA_{5\%}} \ (rest) \end{cases}$$

Hue of VISTA composite image may be determined based on inverse of saturation time. Defining minimum and maximum value as $v_{min}$ and $v_{max}$, hue is determined as

$$\text{hue} = \begin{cases} 0 \ \left(\dfrac{1}{T_{saturation}} > v_{max}\right) \\ 240 \ \left(\dfrac{1}{T_{saturation}} < v_{min}\right) \\ 240 \times \left\{ 1 - \left( \dfrac{\frac{1}{T_{saturation}} - v_{min}}{v_{max} - v_{min}} \right) \right\} \ (rest) \end{cases}$$

Then RGB is determined as

$$\text{if hue} \leq 60; \ R = \text{brightness}, G = \frac{(2R - B)\tan(hue) + \sqrt{3}B}{\tan(hue) + \sqrt{3}}, B = 0$$

$$\text{if } 60 < \text{hue} \leq 120; \ R = \frac{(G + B)\tan(hue) + \sqrt{3}(G - B)}{2\tan(hue)}, G = \text{brightness}, B = 0$$

$$\text{if } 120 < \text{hue} \leq 180; \ R = 0, G = \text{brightness}, B = \frac{(2R - G)\tan(hue) - \sqrt{3}G}{\tan(hue) - \sqrt{3}}$$

$$\text{if } 180 < \text{hue} \leq 240; \ R = 0, G = \frac{(2R - B)\tan(hue) + \sqrt{3}B}{\tan(hue) + \sqrt{3}}, B = \text{brightness}$$

Thus, red, green, and blue areas correspond to high, moderate, and slow flow in a VISTA composite image.

As used herein, an element or step recited in the singular and proceeded with the word "a" or "an" should be understood as not excluding plural elements or steps, unless such exclusion is explicitly recited. Furthermore, references to "one embodiment" of the present invention are not intended to be interpreted as excluding the existence of additional embodiments that also incorporate the recited features.

Control and processing methods and/or systems described herein may be implemented using computer programming or engineering techniques including computer software, firmware, hardware or any combination or subset thereof, wherein the technical effects may include at least processing of the three-dimensional data and quantitative values according to the present disclosure.

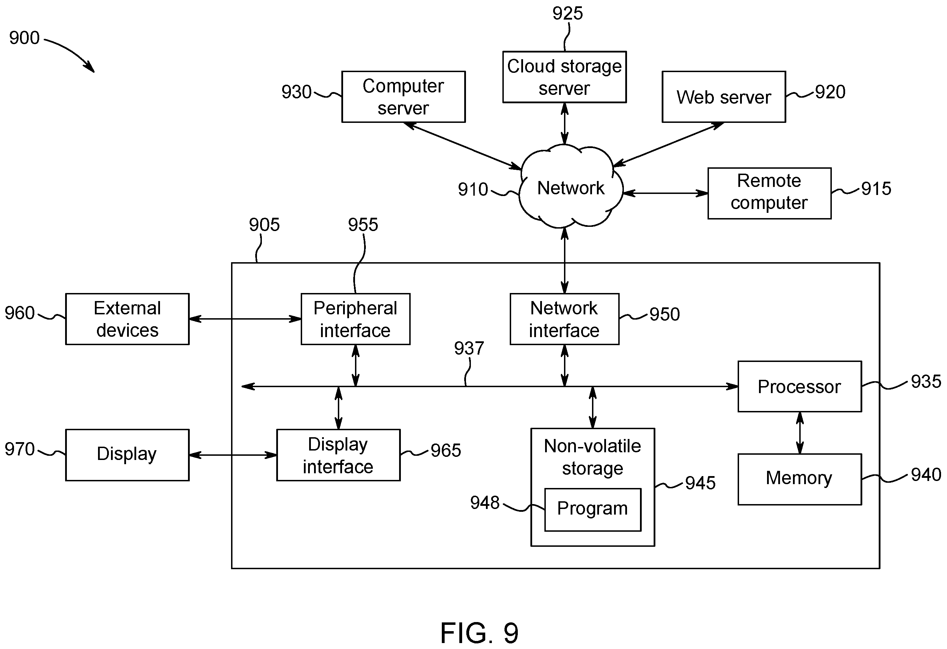

FIG. 9 illustrates a block diagram of a computer that may implement the various embodiments described herein, including the controller 102 and the analyzer 104. Control and processing aspects of the present disclosure may be embodied as a system, a method, and/or a computer program product. The computer program product may include a computer readable storage medium on which computer readable program instructions are recorded that may cause one or more processors to carry out aspects of the embodiment.

The computer readable storage medium may be a tangible and non-transitory device that can store instructions for use by an instruction execution device (processor). The computer readable storage medium may be, for example, but is not limited to, an electronic storage device, a magnetic storage device, an optical storage device, an electromagnetic storage device, a semiconductor storage device, or any appropriate combination of these devices. A non-exhaustive list of more specific examples of the computer readable storage medium includes each of the following (and appropriate combinations): flexible disk, hard disk, solid-state drive (SSD), random access memory (RAM), read-only memory (ROM), erasable programmable read-only memory (EPROM or Flash), static random access memory (SRAM), compact disc (CD or CD-ROM), digital versatile disk (DVD), MO, and memory card or stick. A computer readable storage medium, as used in this disclosure, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions implementing the functions described in this disclosure can be downloaded to an appropriate computing or processing device from a computer readable storage medium or to an external computer or external storage device via a global network (i.e., the Internet), a local area network, a wide area network and/or a wireless network. The network may include copper transmission wires, optical communication fibers, wireless transmission, routers, firewalls, switches, gateway computers and/or edge servers. A network adapter card or network interface in each computing or processing device may receive computer readable program instructions from the network and forward the computer readable program instructions for storage in a computer readable storage medium within the computing or processing device.

Computer readable program instructions for carrying out operations of the present disclosure may include machine language instructions and/or microcode, which may be compiled or interpreted from source code written in any combination of one or more programming languages, including assembly language, Basic, Fortran, Java, Python, R, C, C++, C# or similar programming languages. The computer readable program instructions may execute entirely on a user's personal computer, notebook computer, tablet, or smartphone, entirely on a remote computer or computer server, or any combination of these computing devices. The remote computer or computer server may be connected to the user's device or devices through a computer network, including a local area network or a wide area network, or a global network (i.e., the Internet). In some embodiments, electronic circuitry including, for example, programmable logic circuitry, field-programmable gate arrays (FPGA), or programmable logic arrays (PLA) may execute the computer readable program instructions by using information from the computer readable program instructions to configure or customize the electronic circuitry, in order to perform aspects of the present disclosure.

Aspects of the present disclosure are described herein with reference to flow diagrams and block diagrams of methods, apparatus (systems), and computer program products according to embodiments of the disclosure. It will be understood by those skilled in the art that each block of the flow diagrams and block diagrams, and combinations of blocks in the flow diagrams and block diagrams, can be implemented by computer readable program instructions.

The computer readable program instructions that may implement the systems and methods described in this disclosure may be provided to one or more processors (and/or one or more cores within a processor) of a general purpose computer, special purpose computer, or other programmable apparatus to produce a machine, such that the instructions, which execute via the processor of the computer or other programmable apparatus, create a system for implementing the functions specified in the flow diagrams and block diagrams in the present disclosure. These computer readable program instructions may also be stored in a computer readable storage medium that can direct a computer, a programmable apparatus, and/or other devices to function in a particular manner, such that the computer readable storage medium having stored instructions is an article of manufacture including instructions which implement aspects of the functions specified in the flow diagrams and block diagrams in the present disclosure.

The computer readable program instructions may also be loaded onto a computer, other programmable apparatus, or other device to cause a series of operational steps to be performed on the computer, other programmable apparatus or other device to produce a computer implemented process, such that the instructions which execute on the computer, other programmable apparatus, or other device implement the functions specified in the flow diagrams and block diagrams in the present disclosure.

FIG. 9 is a functional block diagram illustrating a networked system 900 of one or more networked computers and servers. In an embodiment, the hardware and software environment illustrated in FIG. 9 may provide an exemplary platform for implementation of the software and/or methods according to the present disclosure. Referring to FIG. 9, a networked system 900 may include, but is not limited to, computer 905, network 910, remote computer 915, web server 920, cloud storage server 925 and computer server 930. In some embodiments, multiple instances of one or more of the functional blocks illustrated in FIG. 9 may be employed.

Additional detail of a computer 905 is also shown in FIG. 9. The functional blocks illustrated within computer 905 are provided only to establish exemplary functionality and are not intended to be exhaustive. And while details are not provided for remote computer 915, web server 920, cloud storage server 925 and computer server 930, these other computers and devices may include similar functionality to that shown for computer 905. Computer 905 may be a personal computer (PC), a desktop computer, laptop computer, tablet computer, netbook computer, a personal digital assistant (PDA), a smart phone, or any other programmable electronic device capable of communicating with other devices on network 910.

Computer 905 may include processor 935, bus 937, memory 940, non-volatile storage 945, network interface 950, peripheral interface 955 and display interface 965. Each of these functions may be implemented, in some embodiments, as individual electronic subsystems (integrated circuit chip or combination of chips and associated devices), or, in other embodiments, some combination of functions may be implemented on a single chip (sometimes called a system on chip or SoC).

Processor 935 may be one or more single or multi-chip microprocessors, such as those designed and/or manufactured by Intel Corporation, Advanced Micro Devices, Inc. (AMD), Arm Holdings (Arm), Apple Computer, etc. Examples of microprocessors include Celeron, Pentium, Core i3, Core i5 and Core i7 from Intel Corporation; Opteron, Phenom, Athlon, Turion and Ryzen from AMD; and Cortex-A, Cortex-R and Cortex-M from Arm. Bus 937 may be a proprietary or industry standard high-speed parallel or serial peripheral interconnect bus, such as ISA, PCI, PCI Express (PCI-e), AGP, and the like.

Memory 940 and non-volatile storage 945 may be computer-readable storage media. Memory 940 may include any suitable volatile storage devices such as Dynamic Random Access Memory (DRAM) and Static Random Access Memory (SRAM). Non-volatile storage 945 may include one or more of the following: flexible disk, hard disk, solid-state drive (SSD), read-only memory (ROM), erasable programmable read-only memory (EPROM or Flash), compact disc (CD or CD-ROM), digital versatile disk (DVD) and memory card or stick.

Program 948 may be a collection of machine readable instructions and/or data that is stored in non-volatile storage 945 and is used to create, manage and control certain software functions that are discussed in detail elsewhere in the present disclosure and illustrated in the drawings. In some embodiments, memory 940 may be considerably faster than non-volatile storage 945. In such embodiments, program 948 may be transferred from non-volatile storage 945 to memory 940 prior to execution by processor 935.

Computer 905 may be capable of communicating and interacting with other computers via network 910 through network interface 950. Network 910 may be, for example, a local area network (LAN), a wide area network (WAN) such as the Internet, or a combination of the two, and may include wired, wireless, or fiber optic connections. In general, network 910 can be any combination of connections and protocols that support communications between two or more computers and related devices.

Peripheral interface 955 may allow for input and output of data with other devices that may be connected locally with computer 905. For example, peripheral interface 955 may provide a connection to external devices 960. External devices 960 may include devices such as a keyboard, a mouse, a keypad, a touch screen, and/or other suitable input devices. External devices 960 may also include portable computer-readable storage media such as, for example, thumb drives, portable optical or magnetic disks, and memory cards. Software and data used to practice embodiments of the present disclosure, for example, program 948, may be stored on such portable computer-readable storage media. In such embodiments, software may be loaded onto non-volatile storage 945 or, alternatively, directly into memory 940 via peripheral interface 955. Peripheral interface 955 may use an industry standard connection, such as RS-232 or Universal Serial Bus (USB), to connect with external devices 960.

Display interface 965 may connect computer 905 to display 970. Display 970 may be used, in some embodiments, to present a command line or graphical user interface to a user of computer 905. Display interface 965 may connect to display 970 using one or more proprietary or industry standard connections, such as VGA, DVI, DisplayPort and HDMI.

As described above, network interface 950, provides for communications with other computing and storage systems or devices external to computer 905. Software programs and data discussed herein may be downloaded from, for example, remote computer 915, web server 920, cloud storage server 925 and computer server 930 to non-volatile storage 945 through network interface 950 and network 910. Furthermore, the systems and methods described in this disclosure may be executed by one or more computers connected to computer 905 through network interface 950 and network 910. For example, in some embodiments the systems and methods described in this disclosure may be executed by remote computer 915, computer server 930, or a combination of the interconnected computers on network 910.

Data, datasets and/or databases employed in embodiments of the systems and methods described in this disclosure may be stored and or downloaded from remote computer 615, web server 620, cloud storage server 625 and computer server 630.

Numerous modifications and variations of the present invention are possible in light of the above teachings. It is therefore to be understood that within the scope of the appended claims, the invention may be practiced otherwise than as specifically described herein.

**Claims**

1. A scanning apparatus comprising:

   a scanner (104) configured to move a scanning spot along a movement path to capture three-dimensional information of a target object, the movement path including a plurality of b-scan paths (204) performed along a c-scan path (202); and
   a processing circuit configured as a controller (102) to control the movement path of the scanning spot, and

wherein each of the plurality of b-scan paths (204) is a closed loop that returns to a starting point,
wherein the c-scan path (202) is a spiral moving from one of a central region or a peripheral region to the other, or an expanding or contracting rectangular path that moves from one of a central region and a peripheral region to the other.

2. The scanning apparatus according to claim 1, wherein each of the plurality of b-scan paths (204) has a same shape and size.

3. The scanning apparatus according to claim 1, wherein each of the plurality of b-scan paths (204) is a circular path.

4. The scanning apparatus according to claim 1, wherein the controller (102) is further configured to control an inter-scan time by varying a length of each of the b-scan paths (204) and maintaining a sampling step less than a spot size.

5. The scanning apparatus according to claim 1, wherein the controller (102) is further configured to control the b-scan path (204) to move continuously and with a constant speed in each of x and y directions.

6. The scanning apparatus according to claim 1, wherein the controller (102) is further configured to control the scanner (104) to perform multiple b-scan paths (204) in a same location before moving the scanning path to a next position along the c-scan path (202).

7. The scanning apparatus according to claim 1, wherein the b-scan path (204) and the c-scan path (202) are each moved through two or three dimensions.

8. The scanning apparatus according to claim 1, wherein:

a start of a previous b-scan is at a first angular position with respect to a center of the previous b-scan;
a start of a next b-scan is at a second angular position with respect to a center of the next b-scan; and
the first angular position is different than the second angular position.

9. A scanning method comprising:

moving a scanning spot along a movement path to capture three-dimensional information of a target object, the movement path including a plurality of b-scan paths (204) performed along a c-scan path (202); and
controlling the movement path of the scanning spot, and
wherein each of the plurality of b-scan paths (204) is a closed loop that returns to a starting point,
wherein the c-scan path (202) is a spiral moving from one of a central region or a peripheral region to the other, or an expanding or contracting rectangular path that moves from one of a central region and a peripheral region to the other.

10. A computer readable medium storing computer instructions that when executed by a computer of a scanning apparatus according to any one of claims 1-8 perform steps comprising:

controlling movement of a scanning spot along a movement path to capture three-dimensional information of a target object, the movement path including a plurality of b-scan paths (204) performed along a c-scan path (202), and
wherein each of the plurality of b-scan paths (204) is a closed loop that returns to a starting point,
wherein the c-scan path (202) is a spiral moving from one of a central region or a peripheral region to the other, or an expanding or contracting rectangular path that moves from one of a central region and a peripheral region to the other.

**Patentansprüche**

1. Scanvorrichtung, die Folgendes umfasst:

einen Scanner (104), der so konfiguriert ist, dass er einen Scanpunkt eine Bewegungsbahn entlang bewegt und so dreidimensionale Informationen zu einem Zielobjekt erfasst, wobei zu der Bewegungsbahn mehrere B-Scanbahnen (204) gehören, die entlang einer C-Scanbahn (202) vollzogen werden, und

eine Verarbeitungsschaltung, die als Steuerung (102) konfiguriert ist und so die Bewegungsbahn des Scanpunkts steuert, und
wobei es sich bei jeder der mehreren B-Scanbahnen (204) um eine geschlossene Schleife handelt, die zu einem Ausgangspunkt zurückkehrt,
wobei es sich bei der C-Scanbahn (202) um eine Spirale handelt, die sich von einer mittleren Region zu einer Umfangsregion bewegt oder umgekehrt, oder um eine sich vergrößernde oder verkleinernde Rechteckbahn, die sich von einer mittleren Region oder einer Umfangsregion zu der jeweils anderen Region hin bewegt.

2. Scanvorrichtung nach Anspruch 1, wobei jede der mehreren B-Scanbahnen (204) die gleiche Form und Größe aufweist.

3. Scanvorrichtung nach Anspruch 1, wobei es sich bei jeder der mehreren B-Scanbahnen (204) um eine Kreisbahn handelt.

4. Scanvorrichtung nach Anspruch 1, wobei die Steuerung (102) ferner so konfiguriert ist, dass sie einen Zeitraum zwischen Scans regelt, indem sie eine Länge jeder der B-Scanbahnen (204) variiert und einen Abtastschritt beibehält, der kürzer ist als eine Punktgröße.

5. Scanvorrichtung nach Anspruch 1, wobei die Steuerung (102) ferner so konfiguriert ist, dass sie die B-Scanbahn (204) so steuert, dass sie sich ständig und mit einer konstanten Geschwindigkeit in x- und y-Richtung bewegt.

6. Scanvorrichtung nach Anspruch 1, wobei die Steuerung (102) ferner so konfiguriert ist, dass sie den Scanner (104) so steuert, dass er an der gleichen Stelle mehrere B-Scanbahnen (204) vollzieht, bevor er die Scanbahn in eine nächste Position entlang der C-Scanbahn (202) bewegt.

7. Scanvorrichtung nach Anspruch 1, wobei sich die B-Scanbahn (204) und die C-Scanbahn (202) jeweils in zwei oder drei Dimensionen bewegen.

8. Scanvorrichtung nach Anspruch 1, wobei:

sich ein Anfangspunkt eines vorherigen B-Scans in einer ersten Winkelstellung in Bezug auf einen Mittelpunkt des vorherigen B-Scans befindet,
sich ein Anfangspunkt eines nächsten B-Scans in einer zweiten Winkelstellung in Bezug auf einen Mittelpunkt des nächsten B-Scans befindet und
sich die erste von der zweiten Winkelstellung unterscheidet.

9. Scanverfahren, das Folgendes umfasst:

Bewegen eines Scanpunkts entlang einer Scanbahn zwecks Erfassens von dreidimensionalen Informationen zu einem Zielobjekt, wobei zu der Bewegungsbahn mehrere B-Scanbahnen (204) gehören, die entlang einer C-Scanbahn (202) vollzogen werden, und
Steuern der Bewegungsbahn des Scanpunkts und
wobei es sich bei jeder der mehreren B-Scanbahnen (204) um eine geschlossene Schleife handelt, die zu einem Ausgangspunkt zurückkehrt,
wobei es sich bei der C-Scanbahn (202) um eine Spirale handelt, die sich von einer mittleren Region zu einer Umfangsregion bewegt oder umgekehrt, oder um eine sich vergrößernde oder verkleinernde Rechteckbahn, die sich von einer mittleren Region zu einer Umfangsregion bewegt oder umgekehrt.

10. Computerlesbares Medium, auf dem Computeranweisungen gespeichert sind, die, wenn sie von einem Computer einer Scanvorrichtung nach einem der Ansprüche 1-8 ausgeführt werden, Schritte ausführen, welche Folgendes umfassen:

Steuern einer Bewegung eines Scanpunkts entlang einer Bewegungsbahn zwecks Erfassens von dreidimensionalen Informationen zu einem Zielobjekt, wobei zu der Bewegungsbahn mehrere B-Scanbahnen (204) gehören, die entlang einer C-Scanbahn (202) vollzogen werden, und
wobei es sich bei jeder der mehreren B-Scanbahnen (204) um eine geschlossene Schleife handelt, die zu einem Ausgangspunkt zurückkehrt,
wobei es sich bei der C-Scanbahn (202) um eine Spirale handelt, die sich von einer mittleren Region zu einer Umfangsregion bewegt oder umgekehrt, oder um eine sich vergrößernde oder verkleinernde Rechteckbahn, die sich von einer mittleren Region zu einer Umfangsregion bewegt oder umgekehrt.

**Revendications**

1. Appareil de balayage comprenant :

   un scanner (104) configuré pour déplacer un point de balayage le long d'une trajectoire de mouvement afin de capturer des informations tridimensionnelles d'un objet cible, la trajectoire de mouvement comprenant une pluralité de trajectoires de balayage b (204) effectuées le long d'une trajectoire de balayage c (202) ; et
   un circuit de traitement configuré comme un contrôleur (102) pour commander la trajectoire de déplacement du point de balayage,
   chacune de la pluralité de trajectoires de balayage b (204) étant une boucle fermée qui retourne à un point de départ,
   la trajectoire de balayage c (202) étant une spirale se déplaçant d'une zone centrale ou d'une zone périphérique à l'autre, ou une trajectoire rectangulaire en expansion ou en contraction qui se déplace d'une zone centrale ou d'une zone périphérique à l'autre.

2. Appareil de balayage selon la revendication 1, dans lequel chacune de la pluralité de trajectoires de balayage b (204) a la même forme et la même taille.

3. Appareil de balayage selon la revendication 1, dans lequel chacune de la pluralité de trajectoires de balayage b (204) est une trajectoire circulaire.

4. Appareil de balayage selon la revendication 1, dans lequel le contrôleur (102) est en outre configuré pour commander un temps inter-balayage en faisant varier une longueur de chacune des trajectoires de balayage b (204) et en maintenant une étape d'échantillonnage inférieure à une taille de point.

5. Appareil de balayage selon la revendication 1, dans lequel le contrôleur (102) est en outre configuré pour commander la trajectoire de balayage b (204) pour qu'elle se déplace de manière continue et à une vitesse constante dans chacune des directions x et y.

6. Appareil de balayage selon la revendication 1, dans lequel le contrôleur (102) est en outre configuré pour commander le scanner (104) pour effectuer de multiples trajectoires de balayage b (204) dans un même emplacement avant de déplacer la trajectoire de balayage vers une position suivante le long de la trajectoire de balayage c (202).

7. Appareil de balayage selon la revendication 1, dans lequel la trajectoire de balayage b (204) et la trajectoire de balayage c (202) sont chacune déplacées à travers deux ou trois dimensions.

8. Appareil de balayage selon la revendication 1, dans lequel :

   le début d'une acquisition b précédente se trouve à une première position angulaire par rapport au centre de l'acquisition b précédente ;
   le début d'un balayage b suivant se trouve à une seconde position angulaire par rapport au centre du balayage b suivant ; et
   la première position angulaire est différente de la seconde position angulaire.

9. Procédé de balayage comprenant :

   le déplacement d'un point de balayage le long d'une trajectoire de mouvement pour capturer des informations tridimensionnelles d'un objet cible, la trajectoire de mouvement comprenant une pluralité de trajectoires de balayage b (204) effectuées le long d'une trajectoire de balayage c (202) ; et
   le contrôle de la trajectoire du point de balayage, et
   chacune de la pluralité de trajectoires de balayage b (204) étant une boucle fermée qui retourne à un point de départ,
   la trajectoire de balayage c (202) étant une spirale se déplaçant d'une zone centrale ou d'une zone périphérique à l'autre, ou une trajectoire rectangulaire en expansion ou en contraction qui se déplace d'une zone centrale ou d'une zone périphérique à l'autre.

10. Support lisible par ordinateur stockant des instructions d'ordinateur qui, lorsqu'elles sont exécutées par un ordinateur d'un appareil de balayage selon l'une quelconque des revendications 1 à 8, exécutent les étapes comprenant

le contrôle du mouvement d'un point de balayage le long d'une trajectoire de mouvement pour capturer des informations tridimensionnelles d'un objet cible, la trajectoire de mouvement comprenant une pluralité de trajectoires de balayage b (204) effectuées le long d'une trajectoire de balayage c (202),
chacune de la pluralité de trajectoires de balayage b (204) étant une boucle fermée qui retourne à un point de départ,
la trajectoire de balayage c (202) étant une spirale se déplaçant d'une zone centrale ou d'une zone périphérique à l'autre, ou une trajectoire rectangulaire en expansion ou en contraction qui se déplace d'une zone centrale ou d'une zone périphérique à l'autre.

102    104    106

| Controller | → | Scanner | → | Analyzer | → Results |

108

FIG. 1

Cd 210

C-Scan 202

B-Scan 204

206 LΔ

208 Bd

y

x

FIG. 2A

FIG. 2B

FIG. 3A

FIG. 3B

FIG. 4A

FIG. 4B

FIG. 5

602

604

FIG. 6A

FIG. 6B

**FIG. 7**

**FIG. 8**

FIG. 9

1002       1004       1006       1008

| Controller | → | Scanner | → | Analyzer | → Results |

## FIG. 10

B-Scan
1102

C-Scan
1104

## FIG. 11A

B-Scan
1102

Flyback
1106

C-Scan
1104

FIG. 11B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017105618 A1
- EP 3021071 A1
- US 2021109340 A1